# EUROPÄISCHE PATENTSCHRIFT

(11) **EP 1 654 988 B1**
(45) Veröffentlichungstag und Bekanntmachung des Hinweises auf die Patenterteilung: **17.12.2008**
(21) Anmeldenummer: 05023512.6
(22) Anmeldetag: 27.10.2005
(51) Int. Cl.: A61B 17/04, A61F 2/08

(54) **Stift zum Fixieren eines unter Zuglast beanspruchten Implantates**
Pin for the fixation of an implant under load
Broche pour la fixation d'un implant sous contraintes

(30) Priorität: 03.11.2004 DE 102004053471
(43) Veröffentlichungstag der Anmeldung: 10.05.2006
(73) Patentinhaber: Karl Storz GmbH & Co. KG, 78532 Tuttlingen (DE)
(72) Erfinder: Berberich, Sascha, 78532 Tuttlingen (DE)
(74) Vertreter: Weller, Wolfgang

(56) Entgegenhaltungen:
- EP-A- 1 180 351
- EP-A- 1 297 799
- WO-A-02/091928
- WO-A-20/04062459
- AU-B2- 767 853
- US-A- 5 480 403
- US-A1- 2001 053 934
- US-B1- 6 280 474

## Beschreibung

Die Erfindung betrifft einen Stift zum Fixieren eines unter Zuglast beanspruchten Implantates, insbesondere eines Sehnenimplantates, mit einem stabförmigen Körper, auf dessen Querschnitt auf einer Seite die durch das Implantat ausgeübte Kraft einleitbar und auf der gegenüberliegenden Seite in eine Verankerungsstelle ableitbar ist, wobei der Querschnitt auf der krafteinleitenden Seite ein kreisbogenförmiges Profil und auf der kraftableitenden Seite zumindest zwei Auflagestellen aufweist, über die die eingeleitete Kraft verteilt ableitbar ist.

Ein derartiger Stift, auch Cross-Pin genannt, ist aus der US-A-2001/0053934 bekannt.

Derartige Stifte dienen dazu, ein Implantat, das in einer Öffnung in einem Knochen eingesetzt ist, zu fixieren. Dazu wird zunächst das Implantat in die Öffnung, meist eine von der Außenseite her bewerkstelligte Bohrung, eingeschoben. Anschließend wird der Stift quer dazu eingetrieben, dabei die Bohrung und das darin eingeschobene Implantat durchquerend, wodurch dieses in der Lage fixiert ist. Da der Stift quer zur Längserstreckung des Implantates verläuft, hat sich der Ausdruck "Cross Pin" etabliert.

Eine weitverbreitete Anwendung ist die Fixierung eines Implantates, das als Ersatz der Kreuzbänder im Knie dient.

Beim Ersatz des Kreuzbandes hat sich eine Operationstechnik entwickelt, bei der das Implantat bzw. Transplantat, meistens eine Sehne des Patienten, zu einer Schleife gelegt wird und der Cross Pin im Bereich der Schleife quer durchgetrieben wird. Es ist auch bekannt, zwei lose Enden von Sehnenabschnitten miteinander zu vernähen.

Die Sehne umschlingt den stabförmigen Körper in einer Querschnittsebene. Daraus resultiert, dass die von der Sehne ausgeübte Zuglast auf einer Querschnittsseite in den stabförmigen Körper eingeleitet wird und auf der gegenüberliegenden Seite in eine Verankerungsstelle ableitbar ist. Diese Verankerungsstelle stellt die Innenwand, geometrisch gesehen eine Mantellinie der Wand, einer Bohrung dar, in der der Cross Pin aufgenommen ist.

Üblicherweise haben solche Stifte einen kreisförmigen Querschnitt, so dass die kraftableitende Stelle längs einer Mantellinie des stabförmigen Körpers liegt, die der Schleife bzw. der Querschnittsseite gegenüberliegt, um die die Schleife geführt ist.

Dies führt im Belastungsfall dazu, dass die durch das Sehnenimplantat eingeleitete Kraft gegenüberliegend an einer mehr oder weniger begrenzten Stelle in die Verankerungsstelle abgeleitet wird.

Es wurde durch eine Studie festgestellt, dass es bei dieser Geometrie zu einem Bruch des Cross Pins kommen kann.

Um dem abzuhelfen, wurde versucht, in Richtung der Krafteinwirkung unter dem Cross Pin unmittelbar benachbart noch einen zweiten gleichen Cross Pin mit ebenfalls kreisförmigem Querschnitt einzubringen.

Eine weitere Studie hat nun gezeigt, dass auch das Setzen eines zweiten benachbarten Cross Pins einen Bruch nicht ausschließen kann, ganz im Gegenteil, wenn der erste Cross Pin bei einer starken Zugbelastung bricht, so bricht im Anschluss daran auch gleich der zweite Cross Pin; es ist quasi eine Art Dominoeffekt vorhanden.

Aus der WO 2004 / 062459 A sind Fadenanker bekannt, die dazu dienen, in einen Knochen eingetrieben zu werden und einen an dem Anker befestigten Faden zu fixieren. Die Anker weisen an ihrer Außenseite hakenartige Vorsprünge auf, um sich in dem Knochen zu verankern. Es sind unterschiedliche Querschnittsprofile beschrieben, die rund, ovalär, rechteckig, dreieckig oder halbkreisrund sein können.

Aus der US-A-5 480 403 ist ein weiterer Nahtanker für einen Faden bekannt, der eine Spitze zum Eintreiben in den Knochen aufweist. Der Faden ist durch eine Querbohrung am distalen Ende geführt und schmiegt sich in nach proximal verlaufenden Rillen in die Außenseite des Ankerkörpers ein.

Es ist daher Aufgabe der vorliegenden Erfindung, hier Abhilfe zu schaffen und einen Stift bzw. Cross Pin der eingangs genannten Art zum Fixieren eines unter Zuglast beanspruchten Implantates zu schaffen, der hohen Zugbelastungen ohne Bruch widerstehen kann.

Erfindungsgemäß wird die Aufgabe dadurch gelöst, dass der Querschnitt eine grob nierenförmige Form aufweist.

Untersuchungen haben gezeigt, dass bei einer solchen Geometrie die auf der einen Seite von der Sehne eingeleitete Kraft auf die zumindest zwei Auflagestellen verteilt abgeleitet werden kann.

Bei entsprechender symmetrischer Geometrie wird also eine Kraft F, die auf der einen Seite eingeleitet wird, jeweils im Maß F/2 verteilt über die beiden Auflagestellen abgeleitet. Es wurde festgestellt, dass die kraftableitende Stelle ein entscheidendes Kriterium ist, das für den Bruch bei den Cross Pins mit kreisförmigem Querschnitt verantwortlich ist. Mit den nun zumindest zwei Auflagestellen liegt eine Geometrie vor, die bei ansonsten etwa gleichen Außenmaßen eine höhere Bruchbeständigkeit aufweist.

Die beiden im Querschnitt vorhandenen Auflagestellen führen in Längsrichtung des Stiftes gesehen zu entsprechenden, sich über dessen gesamte Länge erstreckenden Stegen, so dass dann über die gesamte Länge des Körpers des Stiftes bzw. Cross Pins gesehen die entsprechenden mehreren Auflagestellen zur Kraftableitung vorhanden sind.

Eine grob nierenförmige Form hat den Vorteil, dass insgesamt ein mechanisch sehr stabiler und kompakter Körper vorhanden ist, der auf einer Seite, also dem Rücken der Niere, eine entsprechend große Anlagefläche für das Implantat bietet, also der Seite, auf der die Kraft einwirkt.

In einer weiteren Ausgestaltung der Erfindung sind die Auflagestellen als Querschnittsvorsprünge ausgebildet.

Diese Maßnahme hat den Vorteil, dass bei dieser Ausgestaltung entsprechende Querschnitte, in Längsrichtung gesehen, entsprechende Stege oder Wülste vorstehen, die für den jeweiligen Einsatzfall eine geeignete Geometrie und eine günstige Anzahl aufweisen können, um bei einer möglichst schlanken Geometrie des stabförmigen Körpers optimale Kraftableitestellen zu bilden, die zur Bruchstabilität beitragen.

Es ist ja nach wie vor das Bestreben, den Cross Pin mit einem möglichst geringen Querschnittsmaß auszugestalten, um den Querschnitt der notwendigen Bohrungen in dem Knochen, in dem der Cross Pin eingetrieben werden soll, möglichst gering zu halten. Dies dient nicht nur dazu, den Knochen möglichst wenig zu schwächen, sondern sorgt auch für ein möglichst rasches Einwachsen des Pins und dient dazu, bei resorbierbaren Materialien auch möglichst wenig Hohlraum zu bilden, der durch eine Resorption des Materials entsteht.

In einer weiteren Ausgestaltung der Erfindung sind die Querschnittvorsprünge abgerundet.

Diese Maßnahme hat den Vorteil, dass die Querschnitte über einen relativ großen Umfangsabschnitt mit der Verankerungsstelle, meist einer Knochenfläche einer Bohrung, in Eingriff treten können, was durch die Abrundung auch besonders atraumatisch erfolgt.

In einer weiteren Ausgestaltung der Erfindung sind drei Auflagestellen vorhanden.

Diese Maßnahme hat den Vorteil, dass über die drei Auflagestellen die einwirkende Kraft gedrittelt abgeleitet werden kann, was zu einer besonders hohen Bruchfestigkeit führt.

In einer weiteren Ausgestaltung der Erfindung weist der Querschnitt in Richtung der Krafteinteilung ein größeres Längenmaß auf als quer zur Längsachse des Stiftes.

Diese Maßnahme führt zu ovalären Querschnitten, deren längere Achse in Richtung der Krafteinleitung verläuft. Dies trägt zusätzlich zur mechanischen Stabilität und damit zur Bruchfestigkeit bei.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele im Zusammenhang mit den beiliegenden Zeichnungen näher beschrieben und erläutert. Es zeigen:
- Fig. 1: einen Stift bzw. Cross Pin in Seitenansicht;
- Fig. 2: einen Schnitt längs der Linie II-II in Fig. 1;
- Fig. 3: in einem etwas vergrößerten Maßstab einen der Fig. 2 entsprechenden Schnitt eines Beispiels mit drei Auflagestellen und ovalärer Form, wobei ein Sehnenimplantat, das durch den Stift fixiert werden soll, angedeutet ist; und
- Fig. 4: stark schematisiert einen in einen Oberschenkelknochen eingesetzten Stift von Fig. 1 zur Transfixation eines unter Zuglast beanspruchten Implantates.

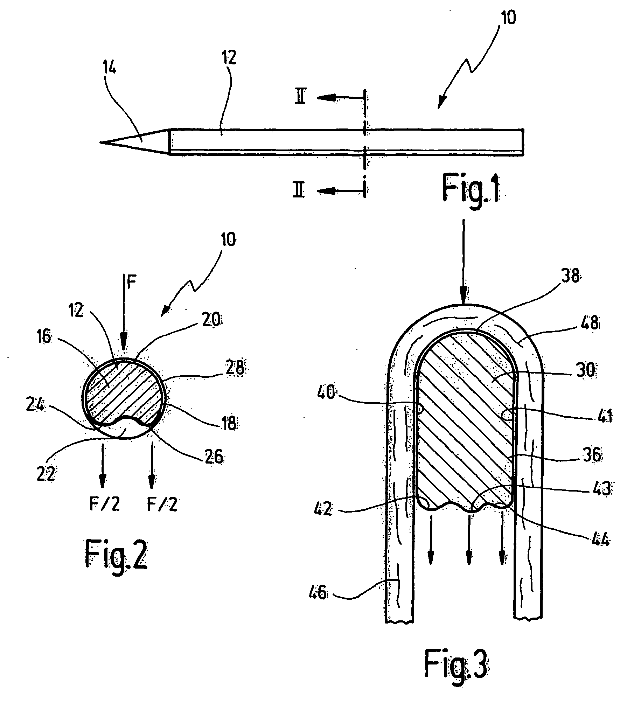

Ein in Fig. 1 und 2 dargestellter Stift bzw. Cross Pin 10 weist einen stabförmigen Körper 12 auf, der an einer Seite in einer konisch zulaufenden Spitze 14 mündet.

Aus der Darstellung von Fig. 2 ist ersichtlich, dass der stabförmige Körper 12 einen Querschnitt 16 aufweist, der grob die Form einer Niere 18 aufweist. Die Umfangskontur ist so, dass eine Seite 20 kreisbogenförmig ist und auf der gegenüberliegenden Seite 22 zwei bogenförmige Auflagestellen 24 und 26 gebildet sind.

In Längsrichtung des stabförmigen Körpers 12 gesehen kann man das so ausdrücken, dass eine Einsenkung oder Auskehlung bzw. Furche ausgespart ist. Der Cross Pin 10 weist beispielsweise eine Länge von 40 mm und einen Durchmesser von etwa 4 mm auf.

Der Stift kann aus resorbierbaren Materialien, aus Kunststoffmaterial oder aus Metall, beispielsweise Titan oder einer Titanlegierung, hergestellt sein.

In Fig. 2 ist dargestellt, wie der Stift 10 etwa passend in eine Bohrung 28 mit kreisförmigem Querschnitt eingesetzt ist.

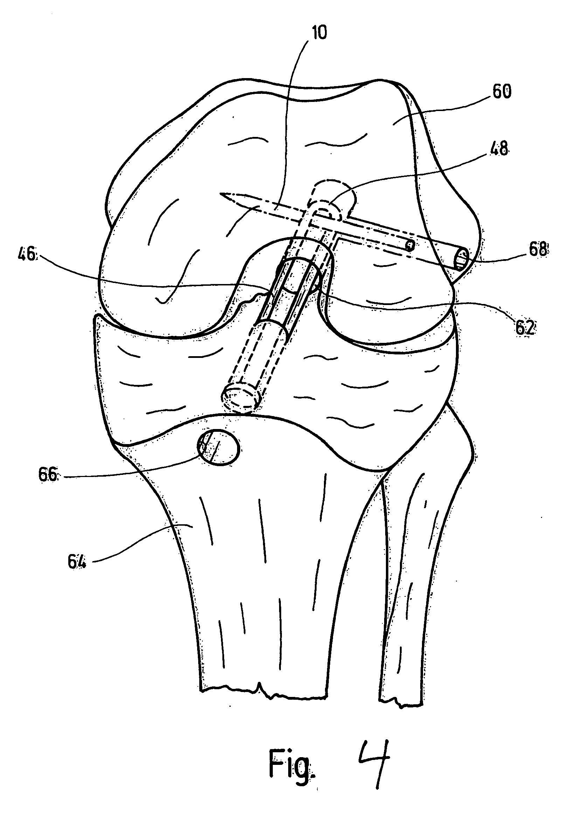

Wirkt nunmehr auf den stabförmigen Körper 12 eine Kraft F ein, und zwar quer zur Längsachse, also in Richtung etwa eines Durchmessers, so ist ersichtlich, dass die Kraft F über die beiden Auflagestellen 24 und 26 jeweils als F/2 in das die Bohrung umgebende Material abgeleitet wird, beispielsweise in ein Knochenmaterial, in das der Stift 10 eingesetzt wird, wie dies später noch im Zusammenhang mit Fig. 4 beschrieben werden wird.

Diese Kraftableitung über zwei Auflagestellen trägt erheblich zur Bruchstabilität bei.

In Fig. 3 ist ein Stift 30 dargestellt, der ebenfalls einen hier nicht näher dargestellten stabförmigen Körper mit einer Spitze aufweist, und der einen Querschnitt 36 aufweist. Der Querschnitt 36 weist auf einer Seite ein halbrundes Profil 38 auf, das über zwei parallele Seiten 40 und 41 an dem dem Halbrund 38 gegenüberliegenden Ende in drei vorspringende Auflagestellen 42, 43 und 44 übergeht.

Es ist dargestellt, wie eine Schleife 48 eines Sehnenimplantates 46 um den Querschnitt 36 gelegt ist, und zwar so, dass die Schleife 48 um das Halbrund 38 gelegt ist.

Im praktischen Einsatz wird das Sehnenimplantat 46 so zugbelastet, dass die Kraft in Richtung des dargestellten Pfeiles einwirkt, also in Richtung vom Halbrund 38 hin zu den drei Auflagestellen 42, 43 und 44.

Dort wird dann von den drei Auflagestellen die Kraft F jeweils gedrittelt abgeleitet.

Ferner ist ersichtlich, dass der Querschnitt 36 in Richtung der Krafteinwirkung ein größeres Längenmaß aufweist als quer zu dieser Richtung, also auch dann quer zur Längsrichtung des stabförmigen Körpers des Stiftes 30. Dies führt nicht nur dazu, wie das aus dem Schnitt von Fig. 3 ersichtlich ist, dass das Sehnenimplantat über einen großen Umschlingungswinkel flächig am Stift 30 anliegt, was ein Ein- und Anwachsen fördert, sondern auch dazu, dass durch diese grob ovaläre Geometrie eine besondere Stabilität in dieser Krafteinwirkungsrichtung bewerkstelligt wird, was letztendlich zusätzlich zur Bruchsicherheit beiträgt.

In Fig. 4 ist eine Situation dargestellt, bei der der Stift 10 bzw. Cross Pin zur Fixation eines Sehnenimplantates 46 dient, das als Ersatz eines Kreuzbandes in dem Knie eines Menschen dient.

Dazu wurde in der Tibia 64 eine durchgehende Bohrung 66 eingebracht, die sich im Femur 60 als Sacklochbohrung 62 weiter fortsetzt.

Ferner wurde im Femur 60 eine Querbohrung 68 eingebracht, um den Stift bzw. Cross Pin 10 eintreiben zu können, und zwar so, dass er durch die Schleife 48 des Sehnenimplantates 46 hindurch reicht. Die Operationstechnik bzw. die notwendigen Geräte zum Einbringen der Bohrungen 62 und 66 sowie zum zielgerechten Einbringen der Bohrung 68 bzw. zum Setzen und Befestigen des Implantates und des Cross Pins sind insbesondere in der US 2003/0065391 A1 und in der US 5,601,562 beschrieben, auf deren Inhalt ausdrücklich diesbezüglich Bezug genommen wird.

Soll ein Cross Pin 30 mit ovalärem Querschnitt eingesetzt werden, wird zunächst mit einem Bohrer die Querbohrung 68 mit rundem Querschnitt bewerkstelligt, und anschließend wird ein Dilatator mit der etwa gleichen Kontur des Cross Pins 30 eingetrieben. Der Dilatator ist etwas kleiner als der Cross Pin 30, so dass beim Einbringen des Cross Pins 30 ein Presssitz entsteht.

## Patentansprüche

1. Stift zum Fixieren eines unter Zuglast beanspruchten Implantates, insbesondere eines Sehnenimplantates (46), mit einem stabförmigen Körper (12), auf dessen Querschnitt (16, 36, 56) auf einer Seite (20) die durch das Implantat (46) ausgeübte Kraft einleitbar und auf der gegenüberliegenden Seite (22) in einer Verankerungsstelle ableitbar ist, wobei der Querschnitt (16, 36, 56) auf der krafteinleitenden Seite (20, 38) ein kreisbogenförmiges Profil und auf der kraftableitenden Seite (22) zumindest zwei Auflagestellen (24, 26) aufweist, über die die eingeleitete Kraft verteilt ableitbar ist, **dadurch gekennzeichnet, dass** der Querschnitt (16) eine grob nierenförmige Form (18) aufweist.

2. Stift nach Anspruch 1, **dadurch gekennzeichnet, dass** die Auflagestellen (24, 26) als Querschnittsvorsprünge ausgebildet sind.

3. Stift nach Anspruch 2, **dadurch gekennzeichnet, dass** die Querschnittsvorsprünge abgerundet sind.

4. Stift nach einem der Ansprüche 1 bis 3, **dadurch gekennzeichnet, dass** drei Auflagestellen vorhanden sind.

5. Stift nach einem der Ansprüche 1 bis 4, **dadurch gekennzeichnet, dass** der Querschnitt in Richtung der Krafteinleitung ein größeres Längenmaß aufweist, als quer zur Längsachse des Stiftes.

## Claims

1. Pin for fixing an implant subjected to tensile load, in particular to a tendon implant (46), said pin having a rod-shaped body (12), the force exerted by the implant (46) being able to be input onto the cross-section (16, 36, 56) of the rod-shaped body on one side (20) and being able to be output on the opposite side (22) to an anchoring side, wherein the cross-section (16, 36, 56) has the shape of an arc of a circle on the force input side (20, 38) and has, on the force output side (22) at least two contact sides (24, 26) via which the input force can be removed in a distributed manner, **characterized in that** the cross-section (26) has a roughly kidney-shaped configuration (18).

2. Pin of claim 1, **characterized in that** the contact sides (24, 76) are designed as cross-section projections.

3. Pin of claim 2, **characterized in that** the cross-section projections are rounded.

4. Pin as claimed in any one of claims 1 through 3, **characterized in that** three contact sides are present.

5. Pin as claimed in any one of claims 1 through 4, **characterized in that** the cross-section has a greater linear dimension in the direction of the force input than it has transverse to the longitudinal axis of the pin.

## Revendications

1. Broche pour la fixation d'un implant soumis à une contrainte de traction, notamment d'un implant tendineux (46), avec un corps en forme de tige (12) sur la section (16, 36, 56) duquel la force exercée par l'implant (26) peut être exercée sur un côté (20) et peut être dissipée dans un point d'ancrage sur le côté opposé (22), sachant que la section (16, 36, 56) présente un profil en arc de cercle sur le côté d'introduction de force (20, 38) et au moins deux points d'appui (24, 26) sur le côté de dissipation de force (22), au moyen desquels la force introduite peut être dissipée d'une manière répartie, **caractérisée en ce que** la section (16) est grossièrement réniforme (18).

2. Broche selon la revendication 1, **caractérisée en ce que** les points d'appui (24, 26) sont réalisés sous la forme de saillies de la section.

3. Broche selon la revendication 2, **caractérisée en ce que** les saillies de la section sont arrondies.

4. Broche selon l'une des revendications 1 à 3, **caractérisée en ce que** trois points d'appui sont présents.

5. Broche selon l'une des revendications 1 à 4, **caractérisée en ce que** la section présente une plus grande longueur dans la direction de l'introduction de force que transversalement à l'axe longitudinal de la broche.
